(19) Europäisches Patentamt
European Patent Office
Office européen des brevets

(11) Publication number: **0 158 847**
**B1**

(12) # EUROPEAN PATENT SPECIFICATION

(45) Date of publication of patent specification: **20.04.88**

(51) Int. Cl.$^4$: **C 07 D 473/32,** C 07 D 473/40, A 61 K 31/52

(21) Application number: **85103162.5**

(22) Date of filing: **13.10.83**

(60) Publication number of the earlier application in accordance with Art. 76 EPC: **0 108 285**

(54) **Antiviral purine derivatives.**

(30) Priority: **14.10.82 US 434393**
**14.10.82 US 434384**
**14.10.82 US 434394**
**14.10.82 US 434395**
**28.07.83 GB 8320309**

(43) Date of publication of application:
**23.10.85 Bulletin 85/43**

(45) Publication of the grant of the patent:
**20.04.88 Bulletin 88/16**

(84) Designated Contracting States:
**AT BE CH DE FR GB IT LI LU NL SE**

(56) References cited:
**EP-A-0 085 424**
**DE-A-2 539 963**

(73) Proprietor: **THE WELLCOME FOUNDATION LIMITED**
**183-193 Euston Road**
**London NW1 2BP (GB)**

(72) Inventor: **Krenitsky, Thomas Anthony**
**106 Laurel Hill Road**
**Chapel Hill North Carolina 27514 (US)**
Inventor: **Beauchamp, Lilia Marie**
**3007 Wycliff Road**
**Raleigh North Carolina 27607 (US)**

(74) Representative: **Dipl.-Ing. Schwabe, Dr. Dr. Sandmair, Dr. Marx**
**Stuntzstrasse 16**
**D-8000 München 80 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to antiviral purine derivatives containing an acyclic chain in the 9-position.

U.K. Patent Specification No. 1523865 describes a broad class of purine derivatives containing an acyclic side chain in the 9-position. These purine derivatives have been found to have antiviral activity against various classes of DNA viruses particularly against herpes viruses such as herpes simplex.

Among these derivatives, 9-(2-hydroxyethoxymethyl)quanine (otherwise known as acyclovir) has been found to have particularly good activity against herpes viruses such as herpes simplex. However, while acyclovir has been found to be especially effective upon topical or parenteral administration, it is only moderately well absorbed upon oral administration with corresponding levels of drug in the plasma. It will be appreciated that when one is treating an internal disorder by oral administration of a drug, it is clearly desirable that the drug should be well absorbed from the gastro-intestinal tract with resulting high plasma levels.

We have now made the surprising discovery that purine derivatives which are characterised by the presence of a hydrogen atom in the 6-position of the purine nucleus, can be readily converted *in vivo* by the action of enzymes of the molybdo-flavo-protein type (especially xanthine oxidase/dehydrogenase or aldehyde oxidase) into the corresponding 6-hydroxy purine derivatives having antiviral activity. Furthermore, from experiments in rats, we have found that oral administration of such 6-hydrogen derivatives results in efficient absorption from the gastro-intestinal tract and high plasma levels of the corresponding 6-hydroxy compound, formed by enzymatic conversion of the 6-hydrogen compound.

The above-mentioned class of 6-hydrogen purine derivatives may be represented by the general formula

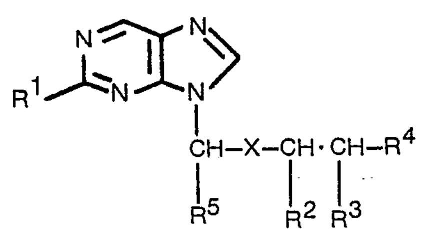

(I)

(wherein X is sulphur or oxygen or a chemical bond; $R^1$ is halogen, amino or azido; $R^2$ is hydrogen, hydroxy, alkyl or hydroxyalkyl; $R^3$ is hydrogen or alkyl; $R^4$ is hydroxy, hydroxyalkyl, benzyloxy, phosphate or carboxypropionyloxy; $R^5$ is hydrogen, alkyl or hydroxyalkyl; and esters of those compounds wherein $R^2$ is hydroxy or hydroxyalkyl, $R^4$ is hydroxy, hydroxyalkyl or carboxypropionyloxy or $R^5$ is hydroxyalkyl; and physiologically acceptable salts of such compounds of formula (I) and their esters, other than (a) the compound of formula (I) wherein X is oxygen, $R^1$ is amino, $R^2$, $R^3$ and $R^5$ are each hydrogen and $R^4$ is hydroxy, and the benzoate ester and physiologically acceptable salts of said compound and (b) the compound of formula (I) wherein X is oxygen, $R^1$ is amino, $R^2$ is hydroxymethyl, $R^3$ and $R^5$ are each hydrogen and $R^4$ is hydroxy, and physiologically acceptable salts and esters of said compound.

In the above formula, the alkyl groups, which may be straight chain or branched, contain 1 to 8, preferably 1 to 4, carbon atoms, e.g. methyl. A preferred class of compounds of formula (I) are those of formula

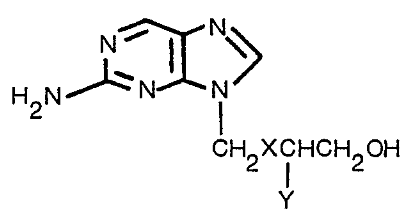

(IA)

(wherein X represents an oxygen or sulphur atom and Y represents a hydrogen atom or a hydroxymethyl group) and physiologically acceptable esters and salts thereof, other than the compound of formula (IA) wherein X represents an oxygen atom and Y represents a hydrogen atom, its benzoate ester and its physiologically acceptable salts.

Salts of the compounds of formula (I) which may be conveniently used in therapy include physiologically acceptable salts of organic acids such as lactic, acetic, malic or p-toluenesulphonic acid as well as physiologically acceptable salts of mineral acids such as hydrochloric or sulphric acid.

Esters of the compounds of formula (I) which may be conveniently used in therapy include those containing a formyloxy or $C_{1-16}$ (for example $C_{1-16}$)alkanoyloxy (e.g. acetoxy or propionyloxy), optionally substituted aralkanoyloxy (e.g. phenyl-$C_{1-14}$alkanoyloxy such as phenyl-acetoxy) or optionally substituted aroyloxy (e.g benzyloxy or naphthoyloxy) ester grouping at one or both in the terminal positions of the 9-

side chain of the compounds of formula (I). The above-mentioned aralkanoyloxy and aroyloxy ester groups may be substituted, for example by one or more halogen (e.g. chlorine or bromine) atoms or amino, nitrile or sulphamido groups, the aryl moiety of the grouping advantageously containing 6 to 10 carbon atoms.

2-Amino-9-(2-benzoyloxyethoxymethyl)purine (i.e. the benzoate ester of the compound of formula (IA) wherein X represents an oxygen atom and Y represents a hydrogen atom) is disclosed in U.S. Patent Specification No. 4,199,574 and no claim is made herein to this compound *per se.* However, there is no indication or suggestion in the said U.S. Specification that the compound can be converted *in vivo* into the corresponding 6-hydroxy analogue.

The discovery that the 6-hydrogen purines above can be readily converted into their corresponding 6-hydroxy analogues is surprising since in previous studies with xanthine oxidase from bovine milk (H.Lettre et al (1967) Biochem.Pharmacol., *16,* 1747—1755; T. A. Krenitsky et al (1972) Arch. Biophys., *150,* 585—599), it was shown that 9-substitution obliterates or greatly diminishes the rate at which a variety of purines are oxidised. In view of these observations, it was surprising to find that this enzyme oxidised for example 6-deoxyacyclovir, a 9-substituted derivative of 2-aminopurine, at a faster rate than the 9-unsubstituted purine, as we have established from enzyme studies.

The above-defined compounds of formula (I) do not include the compound 2-amino-9-(2-hydroxy-1-hydroxymethylethoxy)-methyl-9H-purine, disclosed in European patent specification No. 85 424. However, the following discussion of the medical usages of the compounds of formula (I) also relates to the said compound.

The high level of absorption of the compounds of formula (I) from the gastro-intestinal tract renders the compounds especially useful when oral administration of the compounds is desired, e.g. in the treatment of diseases caused by various DNA viruses, such as herpes infections for example herpes simplex, varicella or zoster, cytomegalovirus as well as diseases caused by hepatitis B or Epstein-Barr virus. The compounds of formula (I) can also be used for the treatment or prophylaxis of papilloma or wart virus infections. In addition to their use in human medical therapy the compounds of formula (I) can be administered to other animals for the treatment of prophylaxis of viral diseases, e.g. in other mammals. For example, those compounds of formula (I) wherein Y represents a hydroxymethyl are especially useful for the treatment of equine rhinopneumonitis. By administration of a compound of formula (I) or physiologically acceptable salt or ester thereof, especially by the oral route, it is possible to achieve an advantageous effect against such disorders.

The compounds of formula (I) and the physiologically acceptable salts and esters thereof (hereafter collectively referred to as the active ingredients) may be administered by any route appropriate to the condition to be treated, suitable routes including oral, rectal, nasal, topical (including buccal and sublingual), vaginal and parenteral (including subcutaneous, intramuscular, intravenous, intradermal, intrathecal and epidural). It will be appreciated that the preferred route may vary with for example the condition of the recipient.

For each of the above-indicated utilities and indications the amount required of an active ingredient (as above defined) will depend upon a number of factors including the severity of the condition to be treated and the identity of the recipient and will ultimately be at the discretion of the attendant physician or veterinarian. In general however, for each of these utilities and indications, a suitable, effective dose will be in the range 0.1 to 250 mg per kilogram bodyweight of recipient per day, preferably in the range 1 to 100 mg per kilogram bodyweight per day and most preferably in the range 5 to 20 mg per kilogram bodyweight per day; an optimum dose is about 10 mg per kilogram bodyweight per day. (Unless otherwise indicated all weights of active ingredient are calculated as the parent compound of formula (I): for salts and esters thereof the figures would be increased proportionately). The desired dose is preferably presented as two, three, four or more sub-doses administered at appropriate intervals throughout the day. These sub-doses may be administered in unit dosage forms, for example, containing 10 to 1000 mg, preferably 20 to 500 mg and most preferably 100 to 400 mg of active ingredient per unit dosage form.

While it is possible for the active ingredients to be administered alone it is preferable to present them as pharmaceutical formulations. The formulations, both for veterinary and for human use, of the present invention comprise at least one active ingredient, as above defined, together with one or more acceptable carriers therefor and optionally other therapeutic ingredients. The carrier(s) must be "acceptable" in the sense of being compatible with the other ingredients of the formulation and not deleterious to the recipient thereof.

The formulations include those suitable for oral, rectal, nasal, topical (including buccal and sublingual), vaginal or parenteral (including subcutaneous, intramuscular, intravenous, intradermal, intrathecal and epidural) administration. The formulations may conveniently be presented in unit dosage form and may be prepared by any of the methods well known in the art of pharmacy. Such methods include the step of bringing into association the active ingredient with the carrier which constitutes one or more accessory ingredients. In general the formulations are prepared by uniformly and intimately bringing into association the active ingredient with liquid carriers or finely divided solid carriers or both, and then, if necessary, shaping the product.

Formulations of the present invention suitable for oral administration may be presented as discrete units such as capsules, cachets or tablets each containing a predetermined amount of the active ingredient; as a powder or granules; as a solution or a suspension in an aqueous liquid or a non-aqueous liquid; or as

an oil-in-water liquid emulsion or a water-in-oil liquid emulsion. The active ingredient may also be presented as a bolus, electuary or paste.

A tablet may be made by compression or moulding, optionally with one or more accessory ingredients. Compressed tablets may be prepared by compressing in a suitable machine the active ingredient in a free-flowing form such as a powder or granules, optionally mixed with a binder, lubricant, inert diluent, preservative, surface active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein.

For infections of the eye or other external tissues e.g. mouth and skin, the formulations are preferably applied as a topical ointment or cream containing the active ingredient in an amount of, for example, 0.075 to 20% w/w, preferably 0.2 to 15% w/w and most preferably 0.5 to 10% w/w. When formulated in an ointment, the active ingredients may be employed with either a paraffinic or a water-miscible ointment base. Alternatively, the active ingredients may be formulated in a cream with an oil-in-water cream base.

If desired, the aqueous phase of the cream base may include, for example, at least 30% w/w of a polyhydric alcohol, i.e. an alcohol having two or more hydroxyl groups such as propylene glycol, butane 1,3-diol, mannitol, sorbitol, glycerol and polyethylene glycol and mixtures thereof. The topical formulations may desirably include a compound which enhances absorption or penetration of the active ingredient through the skin or other affected areas. Examples of such dermal penetration enhancers include dimethylsulphoxide and relates analogues.

The oily phase of the emulsions of this invention may be constituted from known ingredients in a known manner. While the phase may comprise merely an emulsifier (otherwise known as an emulgent), it desirably comprises a mixture of at least one emulsifier with a fat or an oil or with both a fat and an oil. Preferably, a hydrophilic emulsifier is included together with a lipophilic emulsifier which acts as stabiliser. It is also preferred to include both an oil and a fat. Together, the emulsifier(s) with or without stabiliser(s) make up the so-called emulsifying wax, and the wax together with the oil and/fat make up the so-called emulsifying ointment base which forms the oily dispersed phase of the cream formulations.

Emulgents and emulsion stabilisers suitable for use in the formulation of the present invention include Tween® 60, Span® 80, cetostearyl alcohol, myristyl alcohol, glyceryl mono-stearate and sodium lauryl sulphate.

The choice of suitable oils or fats for the formulation is based on achieving the desired cosmetic properties, since the solubility of the active compound in most oils likely to be used in pharmaceutical emulsion formulations is very low. Thus the cream should preferably be a non-greasy, non-staining and washable product with suitable consistency to avoid leakage from tubes or other containers. Straight or branched chain, mono- or dibasic alkyl esters such as di-isoadipate, isocetyl stearate, propylene glycol diester of coconut fatty acids, isopropyl myristate, decyl oleate, isopropyl palmitate, butyl stearate, 2-ethylhexyl palmitate or a blend of branched chain esters known as Crodamol® CAP may be used, the last three being preferred esters. These may be used alone or in combination depending on the properties required. Alternatively, high melting point lipids such as white soft paraffin and/or liquid paraffin or other mineral oils can be used.

Formulations suitable for topical administration to the eye also include eye drops wherein the active ingredient is dissolved or suspended in a suitable carrier, especially an aqueous solvent for the active ingredient. The active ingredient is preferably present in such formulations in a concentration of 0.5 to 20%, advantageously 0.5 to 10% particularly about 1.5% w/w.

Formulations suitable for topical administration in the mouth include lozenges comprising the active ingredient in a flavoured basis, usually sucrose and acacia or tragacanth; pastilles comprising the active ingredient in an inert basis such as gelatin and glycerin, or sucrose and acacia; and mouthwashes comprising the active ingredient in a suitable liquid carrier.

Formulations for rectal administration may be presented as a suppository with a suitable base comprising for example cocoa butter or a salicylate.

Formulations suitable for nasal administration wherein the carrier is a solid include a coarse powder having a particle size for example in the range 20 to 500 μm which is administered in the manner in which snuff is taken, i.e., by rapid inhalation through the nasal passage from a container of the powder held close up to the nose. Suitable formulations wherein the carrier is a liquid, for administration as for example a nasal spray or as nasal drops, include aqueous or oily solutions of the active ingredient.

Formulations suitable for vaginal administration may be presented as pessaries, tampons, creams, gels, pastes, foams or spray formulations containing in addition to the active ingredient such carriers as are known in the art to be appropriate.

Formulations suitable for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze-dried (lyophilized) condition requiring only the addition of the sterile liquid carrier, for example water for injections, immediately prior to use. Extemporaneous injection solutions and suspensions may be

prepared from sterile powders, granules and tablets of the kind previously described. Preferred unit dosage formulations are those containing a daily dose or unit daily sub-dose, as herein above recited, or an appropriate fraction thereof, of an active ingredient.

It should be understood that in addition to the ingredients particularly mentioned above the formulations of this invention may include other agents conventional in the art having regard to the type of formulation in question, for example those suitable for oral administration may include flavouring agents.

The present invention further provides veterinary compositions comprising at least one active ingredient as above defined together with a veterinary carrier therefor.

Veterinary carriers are materials useful for the purpose of administering the composition and may be solid, liquid or gaseous materials which are otherwise inert or acceptable in the veterinary art and are compatible with the active ingredient. These veterinary compositions may be administered orally, parenterally or by any other desired route.

For oral administration the compositions can be in the form of a tablet, granule, drench, paste, cachet, capsule or feed supplement. Granules may be made by the well known techniques of wet granulation, precompression or slugging. They can be administered to animals in an inert liquid vehicle so as to form a drench, or in a suspension with water or oil base. Preferably further accessory ingredients such as a dispensing agent are included. These formulations preferably contain from 15 to 85% of the active ingredient.

A paste may be formulated by suspensing the active ingredient in a liquid diluent. A stiffening or thickening agent may be included together with a wetting agent or a humectant if the liquid diluent is water. If an emulsion paste is needed then one or more surface active agents should desirably be included. From 25 to 80% by weight of these paste formulations may comprise the active ingredient.

In feed supplements the active ingredient is generally present in large amounts relative to the accessory ingredients, and the supplements may be added directly or after intermediate blending or dilution. Examples of accessory ingredients for such formulations include solid, orally ingestible carriers such as corn meal, soya flour, wheat shorts, soya grits, edible vegetable materials and fermentation residues. The active ingredient is usually incorporated in one or more of the accessory ingredients and intimately and uniformly dispersed by grinding, tumbling or stirring with conventional apparatus. Formulations containing 1 to 90% by weight of the active ingredient are suitable for adding to feeds.

For the treatment of herpes infections in horses, an oral or parenteral dose of from 0.1 to 250 mg per kg body weight per day, preferably from 2 to 100 mg per kg per day may be required. The dose may be split up into discrete units administered at regular intervals during the day, and repeated daily for up to 14 days or until the infection is cleared. For viral infections in other animals the dose may vary depending on the size and metabolism of the animal. The compositions may be administered in unit dosage form, such as a tablet a few times daily in the amount of 10 to 1000 mg per unit dose.

The compounds of formula (I) and their physiologically acceptable salts and esters may be prepared in conventional manner by analogous processes for preparing compounds of similar structure, such as those methods described in U.K. Patent Specification No. 1523865.

The present invention provides a process for the preparation of compounds of formula (I) and physiologically acceptable salts and esters thereof other than (a) the compound of formula (I) wherein X is oxygen, $R^1$ is amino, $R^2$, $R^3$ and $R^5$ are each hydrogen and $R^4$ is hydroxy, and the benzoate ester and physiologically acceptable salts of said compound and (b) the compound of formula (I) wherein X is oxygen, $R^1$ is amino, $R^2$ is hydroxymethyl, $R^3$ and $R^5$ are each hydrogen and $R^4$ is hydroxy, and physiologically acceptable salts and esters of said compound which comprises

a) deblocking a compound of formula

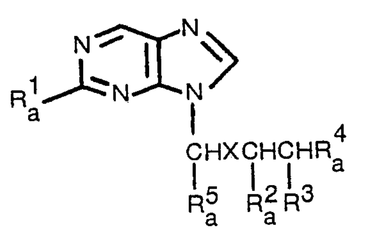

(II)

(wherein X and $R^3$ are as defined above and $R^1_a$ represents an amino or blocked amino group; $R^2_a$ and $R^4_a$, which may be the same or different, each represents a hydroxy or hydroxyalkyl group or a blocked hydroxy or hydroxyalkyl group, $R^5_a$ represents a hydroxyalkyl or blocked hydroxyalkyl group, providing at least one of $R^1_a$, $R^2_a$, $R^4_a$ and $R^5_a$ represents a blocked group);

b) converting a compound of formula

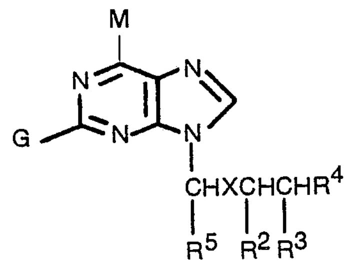

(III)

(wherein X, $R^2$, $R^3$, $R^4$ and $R^5$ are as defined above, M represents a hydrogen atom or a group or atom convertible into a hydrogen atom and G represents a group or atom convertible into an amino group or (when M is other than a hydrogen atom) G may alternatively represent an amino group) or a salt or ester thereof into a compound of formula (I) (wherein $R^1$ represents an amino group) or a physiologically acceptable salt or ester thereof;

c) reacting a compound of formula

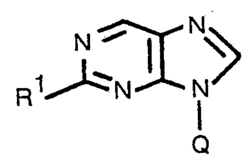

(IV)

(wherein Q represents a leaving atom or group) with a compound of formula

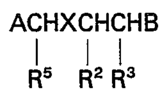

(V)

(wherein X, $R^2$, $R^3$ and $R^5$ are as defined above and A represents a leaving group or atom and B represents an optionally protected hydroxy group);

d) reducing a compound of formula

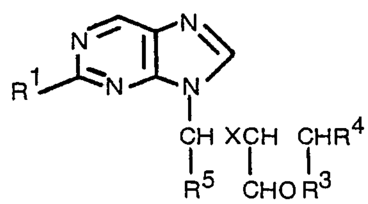

(VI)

(wherein X, $R^1$, $R^3$, $R^4$ and $R^5$ are as defined above) or a salt or ester thereof to form a compound of formula (I) (wherein $R^2$ represents a hydroxymethyl group) or a physiologically acceptable salt or ester thereof;

e) treatment of a compound of formula

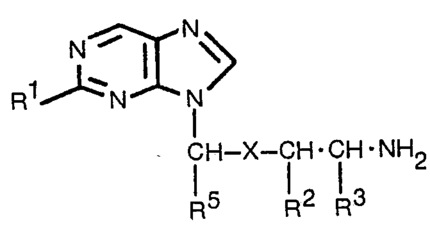

(VII)

(wherein X, $R^1$, $R^2$, $R^3$ and $R^5$ are as defined above) with a nitrite in an acidic medium to form a corresponding compound of formula (I) wherein $R^4$ represents a hydroxy group; and optionally effecting one or more of the following conversions, in any desired sequence:—

i) where the resulting product is a base, converting the said base into a physiologically acceptable acid addition salt thereof;

ii) where the resulting product is an acid addition salt, converting the said salt into the parent base;

iii) where the resulting product is a compound of formula (I) or a salt thereof converting the said compound or salt thereof into a physiologically acceptable ester of the said compound or salt; and/or

iv) where the resulting product is an ester of a compound of formula (I) converting the said ester into the parent compound of formula (I), a physiologically acceptable salt thereof or a different physiologically acceptable ester thereof.

In method a) the blocking groups may be selected, as appropriate, for example from acyl groups such as $C_{1-4}$alkanoyl groups e.g. acetyl or pivaloyl, or aroyl groups, e.g. benzoyl; arylmethyl groups e.g. benzyl; or tri-$C_{1-4}$alkylsilyl e.g. trimethylsilyl. Arylmethyl blocking groups, may be removed for example by hydrogenolysis, e.g. by hydrogenation in the presence of Raney nickel or palladium catalyst or by the use of sodium in liquid ammonia. Acyl blocking groups may be removed for example by hydrolysis using for example an amine such as methylamine or triethylamine, advantageously in an aqueous medium. Trialkylsilyl blocking groups may be removed for example by solvolysis e.g. with alcoholic or aqueous ammonia, or by alcoholysis.

Alternatively, when $R^2_a$ represents a blocked hydroxyalkyl group, $R^3$ represents a hydrogen atom and $R^4_a$ represents a blocked hydroxy group, the blocking may be effected by a common grouping, i.e. as in a compound of formula

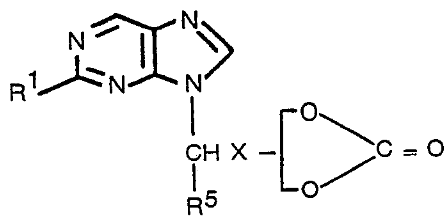

(VIII)

(wherein, X, $R^1$ and $R^5$ are as defined above). Deblocking may be effected, for example, by hydrolysis e.g. under basic conditions, preferably by treatment with an organic amine such as methylamine or triethylamine.

Conversion of a compound of formula (III) into a compound of formula (I), by method b), can be achieved by various conventional means. For example G may represent an azide group which can be reduced to an amino group by catalytic hydrogenation using a suitable catalyst such as palladium. Alternatively, G may represent a halogen atom or an alkylthio or alkylsulphonyl group which can be converted to an amino group by aminolysis using for example ammonia. M may represent a halogen e.g. chlorine, atom or a mercapto or thio (S<) group which can be converted into a hydrogen atom in conventional manner. In the case where M represents a thio group, this conversion may be effected using a compound of formula (III) in which any amino or hydroxyl groups are optionally blocked by acyl groups, the conversion being effected using a Raney nickel catalyst, e.g. in a basic medium which additionally removes the amino and/or hydroxy blocking groups in accordance with process a).

These processes together with other conventional processes are described in Fused Pyrimidines, Part II, Purines Ed. by D. J. Brown (1971), Wiley-Interscience.

In process (c), the group Q in formula (IV) may for example represent a hydrogen atom; an acyl group, e.g. a $C_{1-14}$alkanoyl group such as an acetyl group or an aroyl group such as a benzoyl group; or a tri-$C_{1-14}$alkylsilyl group such as a trimethylsilyl group. The group A in formula (V) may for example represent a halogen atom (e.g. chlorine) or an acyloxy group wherein the acyl moiety may be for example a $C_{1-14}$alkanoyl group such as acetyl, or an aroyl group such as benzoyl. The reaction may be conveniently effected in a strong polar solvent such as dimethylformamide or hexamethylphosphoramide, advantageously in the presence of a base such as triethylamine or potassium carbonate. Alternatively, a thermal condensation may be effected by heating the compounds of formulae (IV) and (V) in the presence of a catalytic amount of a strong acid, e.g. sulphuric acid.

Reduction of a compound of formula (VI) in process (d) may be achieved for example by reaction with an appropriate aldehyde reducing agent such as sodium borohydride, sodium cyanoborohydride, tetraethylammonium borohydride or pyridine/diborane/tetrahydrofuran/trifluoroacetic acid.

In process e) the conversion is advantageously effected by treatment of the amino compound of formula (VII) with a nitrite, e.g. sodium nitrite, in an acidic medium.

The starting materials employed in the processes described above may be prepared in conventional manner, e.g. in accordance with the processes described in the above-mentioned UK Patent Specification 1523865.

The following Examples illustrate the present invention.

## Example 1

2-Amino-9-(2-hydroxyethylthiomethyl)-9H-purine

A 1 litre round bottom flask equipped with a magnetic stirring bar and a $CaCl_2$ drying tube was charged with 5.0 g (29.50 mM) 2-amino-6-chloro-purine, 6.8 g (29.50 mM) 2-(chloromethylthio)ethyl benzoate, 4.07 g (29.50 mM) potassium carbonate and 750 ml of anhydrous dimethyl formamide. The reaction was stirred at room temperature for 16 hours after which time another 2.0 g (8.7 mM) of 2-(chloromethylthio)ethyl benzoate with 1.2 g (8.7 mM) potassium carbonate were added and stirring continued for an additional 24 hours at room temperature. The reaction mixture was then filtered through a pad of Celite® in a sintered glass funnel and rotary evaporated *in vacuo* to a viscous yellow oil.

The oil was adsorbed on to 20.0 g of Merck silica gel 60 (70—230 mesh) and this preadsorbed phase applied to the top of a column of Merck silica gel 60 (230—400 mesh). Elution of the column using 6:4 ethyl

acetate-benzene provided 2-amino-9-(2-benzoyloxyethylthiomethyl)-6-chloro-9H-purine as an off-white solid as a single material by TLC on silica gel 8:2 ethyl acetate-hexane. $^1$H NMR, $CDCl_3$ 8.0—7.15, 6H, multiplet; 5.1, 4H, broad singlet; 4.5, 2H, triplet 2.985, 2H triplet.

The last-mentioned product (0.98 g, 2.70 mM) was placed into a 500 ml Parr hydrogenator bottle together with 2.5 g (Pd $(OH_2)$ catatlyst, 50 ml triethylamine and 200 ml of methanol. This was shaken in a Parr hydrogenator under 3.4 bar (50 p.s.i.) hydrogen for 16 hours at room temperature. TLC of the hydrogenolysis product on silica gel with 8% methanol-ethyl acetate showed only partial reaction. The catalyst was removed by filtration through a pad of Celite® in a sintered glass funnel, 2.0 g of fresh $Pd(OH)_2$ catalyst and 7.0 ml triethylamine were added and the reaction mixture again shaken under 3.4 bar (50 p.s.i.) hydrogen for 20 hours. At this point t.l.c. showed the reaction to be complete, the catalyst was removed as before and the methanol removed by rotary evaporation *in vacuo* to give a clear glass, which was taken up into 100 ml of 1:1 methanol-water containing 20% methylamine. The resulting solution was stirred at room temperature for 4 hours and removed by rotary evaporation *in vacuo* to give an amorphous solid. The solid was adsorbed on to 10.0 g of 70—230 mesh Merck silica gel 60 and this preadsorbed phase applied to the top of a column of 230—400 mesh Merck silica gel 60. Elution of the column using 10% methanol ethyl acetate provided a solid that crystallised from ethyl acetate-hexane to give the title compound in the form of needles, m.p. 122—124°C, t.l.c.: 1 spot on silica gel with 15% methanol:ethyl acetate, $^1$H NMR DMSO-d6 8.59, 1H singlet; 8.15, 1H singlet; 6.56, 2H broad singlet; 5.24, 2H singlet; 4.83, 1H triplet; 3.49, 2H multiplet; 2.69, 2H, multiplet; Anal. Theory C: 42.65, H 4.92, N: 31.09 found C: 42.70, H: 4.94, N: 31.04.

### Example 2

a) 2-Amino-9-[(2-benzoyloxy-1-benzoyloxymethylethoxy)methyl]-2-chloro-9H-purine

A mixture of 7.0 g (41.2 mM) of 2-amino-6-chloro-9H-purine, 4.55 g (34.4 mM) of ammonium sulfate, and 200 ml hexamethyldisilazane were combined under nitrogen and eluted with stirring for five hours. The mixture was flash evaporated, followed by the addition of 8.8 g (23.6 mM) of 2-O (acetylmethyl)-1,3-bis-(O-benzoyl)glycerol in about 10 ml benzene. The mixture was heated for 1.5 hours in an oil bath and under aspirator pressure with a distilling head hookup. Then the mixture was cooled to room temperature. Methanol was added to the mixture which was placed over a steam bath for 30 minutes. The mixture was then washed with water and extracted 3 times with ethyl acetate and the organic layer dried over anhydrous magnesium sulphate for about 30 minutes. The mixture was then filtered, the salt washed with ethyl acetate and then flash evaporated. Flash column chromatography with 6:1 $CH_2Cl_2$/acetone eluent yielded the title compound as an analytically pure white solid with a mpt of 130—131°.

b) 2-Amino-9-[(2-benzoyloxy-1-benzoyloxymethylethoxy)methyl]-9H-purine

A mixture of 3.393 g (7.04 mM) of 2-amino-9-[(2-benzoyloxy-1-benzoyloxymethylethoxy)methyl]-6-chloro-9H-purine, 100 ml ethanol, 100 ml tetrahydrofuran, 1.9 ml triethylamine was added to 0.600 g Pd-C catalyst in a Parr bottle. The mixture was shaken under hydrogen for 24 hours. The Pd-C was filtered through a Celite® pad and 0.650 g fresh Pd-C was added to the reaction mixture and the hydrogenation was continued for 4 days. Column chromatography using 100% $CH_2Cl_2$, 4:1 $CH_2Cl_2$/acetone, and 100% acetone provided the title compound, mpt. 132—133°C.

### Example 3

2-Amino-9-[(2-hydroxy-1-hydroxymethylethoxy)methyl]-9H-purine

A mixture of 0.844 g (1.88 mM) of 2-amino-9-[(2-benzoyloxy-1-benzoyloxymethylethoxy)methyl]-9H-purine and 100 ml of a 40% aqueous methylamine solution was stirred at room temperature for 30 minutes. The mixture was flash evaporated to a yellow oil which was then washed with water. The aqueous layer was then extracted twice with methylene chloride. The aqueous layer containing the product was then flash evaporated to a light yellow oil. Recrystallisation of the oil in hot acetonitrile yielded the title compound as an analytically pure ivory solid mpt. 148—151°.

### Example 4

9-(2-Acetoxyethoxymethyl)-2-amino-9H-purine

A mixture of 0.82 g (3.92 mM) of 9-(2-hydroxyethoxymethyl)-2-amino-9H-purine, 48 mg (0.392 mM) of 4-dimethylaminopyridine and 0.75 ml (7.84 mM) of acetic anhydride in 18 ml of dry dimethylformamide was stirred at room temperature for two days.

The reaction mixture was evaporated *in vacuo* and the residue dissolved in ethyl acetate and absorbed on silica gel. The solvent was removed by flash evaporation and the residual powder added to a column prepared for flash chromatography. Elution with 5% methanol in dichloromethane gave 1.0 g of a semicrystalline oil which on recrystallization from benzene-hexane gave analytically pure 9-(2-acetoxyethoxymethyl)-2-amino-9H-purine, mpt=115—118°C.,

### Example 5

2-Amino-9-[(2-acetoxy-1-acetoxymethylethoxy)methyl]-9H-purine

A mixture of 0.45 g (1.88 mM) of 2-amino-9-[(2-hydroxy-1-hydroxymethylethoxy)methyl]-9H-purine, 1.15 g (11.30 mM) of acetic anhydride and 23 mg (0.188 mM) of 4-dimethylaminopyridine in 10 ml of dry

dimethylformamide was stirred at room temperature for 18 hours. The solution was evaporated *in vacuo* and the residue dissolved in methanol and preabsorbed on silica gel. The solvent was evaporated off *in vacuo* and the powder added to column prepared for flash chromatography. Elution with 10% methanol in dichloromethane yielded on evaporation the title compound. Recrystallization from ethyl acetate-hexane gave analytically pure 2-amino-9-[(2-acetoxy-1-acetoxymethylethoxy)methyl]-9H-purine.

## Example 6

9-(2-Acetoxyethylthiomethyl)-2-amino-9H-purine

A 100 ml flask equipped with a magnetic stirring bar and a $CaCl_2$ drying tube was charged with 1.0 g (4.4 mM) 2-amino-9-(2-hydroxyethylthiomethyl)-9H-purine, 0.054 g (0.44 mM) 4-dimethylaminopyridine, 0.9 ml (8.8 mM) acetic anhydride, and 20 ml of dry DMF. The solution was stirred for 24 hours at room temperature and then quenched with 5 ml of MeOH. The solution was evaporated *in vacuo* (bath temp 40°C) and the brown oil so obtained chromatographed using the "flash" method. Elution of the column with 1.5% methanol in ethyl acetate provided a yellow oil which crystallized on standing. The light yellow crystals were dissolved in a solution of 2% MeOH in toluene and treated with 0.05 g Darco® G-60.

The methanol was evaporated from the toluene solution resulting in the precipitation of light yellow crystals.

The product was dried for 16 hours at 78°C to yield the title compound mp=119—120.5°C. Anal. Calcd. for $C_{10}H_{13}N_5O_2S$: C, 44.93; H, 4.9; N, 26.20. Found: C, 44.97; H, 4.96; N, 26.17. $^1H$ NMR in $DMSO_6$ 8.59 (1HS), 8.15 (1Hs), 6.52 (2H, broad s), 5.26 (2Hs), 4.15 (2Ht J=6.36Hz), 2.89 (2Ht J=6.36Hz), 1.99 (3Hs).

## Example 7

2-Amino-9-(2-benzoyloxyethylthiomethyl)-9H-purine

A 5-litre, 3-neck flask was equipped with an air-stirring motor, glass stirring rod with Teflon® paddle, and provision made for evacuating and filling the flask with either $N_2$ or $H_2$ gas. The flask was then charged with 7.0 g (0.019 M) of 2-amino-9-(2-benzoyloxyethylthiomethyl)-6-chloro-9H-purine, 14.0 g palladium hydroxide on carbon, 12.0 ml (0.163 M) triethylamine, 75.0 ml water and 3.0 litres of methanol. The flask was then sealed and evacuated using a water aspirator, flooded with $N_2$ gas, evacuated until 3 complete cycles were completed. The flask was evacuated, flooded with $H_2$ gas, evacuated and refilled with $H_2$ gas. The stirring motor was started and the reaction was stirred under $H_2$ for 4 days at room temperature.

The solution was filtered through a sintered glass funnel and the catalyst washed with 800 ml of methanol. The methanol solution was evaporated *in vacuo* to give 6.0 g of a light yellow solid. The solid was absorbed onto 18.0 g of 70—230 mesh silica gel 60 and this preabsorbed phase applied to the top of a column of 230—400 mesh silica gel 60. Elution of the column by the "Flash" method using 5% methanol in ethyl acetate provided a white solid which was recrystallized from ethyl acetate-hexane, dried at 78°C under 1.0 mm Hg vacum to white flakes mp 120—121°C, H NMR in DMSO $d_6$ 8.60 (1Hs), 8.19 (1Hs), 8.0 7.5 (5 H,m), 6.55 (2H,s) 5.32 (2H,s), 4.52 (2H,t), 3.06 (2H,t) Anal. theory C: 54.69, H: 4.59 N: 21.26 Anal. found C: 54.68, H: 4.64, N: 21.24.

2-Amino-9-(2-hydroxyethylthiomethyl)-9H-purine was obtained by continued elution of the above column.

The following Examples 8, to 11, illustrate pharmaceutical formulations according to the invention where the active compound is a compound of formula (I) or a physiologically acceptable salt or ester thereof, specifically 2-amino-9-[(2-hydroxy-1-hydroxymethylethoxy)methyl]-9H-purine.

## Example 8

| | Tablet |
|---|---|
| Active compound | 200 mg |
| Lactose | 235 mg |
| Starch | 50 mg |
| Polyvinylpyrrolidone | 50 mg |
| Magnesium stearate | 5 mg |
| | 500 mg |

Mix the active compound with the lactose and starch and wet granulate with a solution of the polyvinylpyrrolidone. Dry, sift, blend and granules with magnesium stearate and compress.

Example 9

| | Capsule |
|---|---|
| Active compound | 200 mg |
| Lactose | 184 mg |
| Sodium starch glycollate | 8 mg |
| Polyvinylpyrrolidone | 6 mg |
| Magnesium stearate | 2 mg |

Mix the active compound with the lactose and sodium starch glycollate and wet granulate with a solution of the polyvinylpyrrolidone. Dry, sift, blend the granules with the magnesium stearate and fill into hard gelatin capsules.

Example 10

| | Cream |
|---|---|
| Active compound | 5.00 g |
| Glycerol | 2.00 g |
| Cetostearyl alcohol | 6.75 g |
| Sodium lauryl sulphate | 0.75 g |
| White soft paraffin | 12.50 g |
| Liquid paraffin | 5.00 g |
| Chlorocresol | 0.10 g |
| Purified water to | 100.00 g |

Dissolve the active compound in a mixture of purified water and glycerol and heat to 70°C. Heat the remaining ingredients together at 70°C. Add the two parts together and emulsify. Cool and fill into containers.

Example 11

Intravenous Injections

| | | | |
|---|---|---|---|
| A) | Active compound | | 200 mg |
| | Sodium hydroxide solution q.s. to pH 7.0 to 7.5 | | |
| | Water for injections | to | 5.0 ml |

Dissolve the active compound in part of the water for injections. Adjust the pH with the sodium hydroxide solution and make up to volume with additional water for injections. Under aseptic conditions, sterilise the solution by filtration, fill into sterile ampoules and seal the ampoules.

| | | | |
|---|---|---|---|
| B) | Active compound | | 100 mg |
| | Sodium hydroxide solution q.s. to pH 7.0 to 7.5 | | |
| | Mannitol | | 125 mg |
| | Water for injections | to | 2.5 ml |

Dissolve the active compound and mannitol in part of the water for injections. Adjust the pH with the sodium hydroxide solution and make up to volume with additional water for injections. Under aseptic conditions, sterilise the solution by filtration, fill into sterile vials and remove the water by freeze-drying. Seal the vials under an atmosphere of nitrogen and close with a sterile stopper and aluminium collar.

Antiviral Activity

40 CD1 mice were infected with 300 $LD_{50}$ of the ICI strain of herpes simplex Type 1 administered in a dose of 0.025 ml intracerebrally. The mice were divided into 4 groups of 10, 1 group was not treated to act as virus controls. Treatment group 1 received 100 mg/kg/dose of the compound of Example 3, treatment group 2 received 100 mg/kg/dose of the compound of Example 2b and treatment group 3 received 100 mg/kg/dose of acyclovir to act as a positive control; all drugs were administered by the subcutaneous route. Survival times were recorded for 14 days and were compared as shown in the following table.

Results

| TREATMENT GROUP | SURVIVAL TIMES (days) | MEAN RECIPROCAL SURVIVAL TIMES |
|---|---|---|
| Virus Control | 1+2 4+2 4+3<br>1+4 | 0.37 |
| Group 1 | 1+9 1+10 8 survivors | 0.02 |
| Group 2 | 1+4 2+4 3+5 | |
| | 1+7 1+8 1+9 | |
| | 1 survivor | 0.17 |
| Group 3 | 1+2 1+3 1+3 | |
| | 1+4 2+5 2+6 | |
| | 1+13 1 survivor | 0.21 |

**Claims**

1. Compounds of the general formula

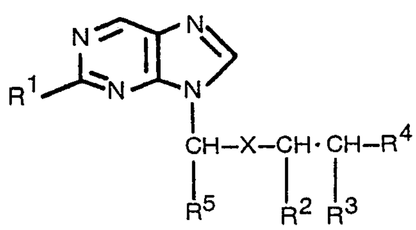

(I)

(wherein X is sulphur or oxygen or a chemical bond; $R^1$ is halogen, amino or azido; $R^2$ is hydrogen, hydroxy, $C_{1-8}$ alkyl or hydroxy-$C_{1-8}$alkyl; $R^3$ is hydrogen or $C_{1-8}$alkyl; $R^4$ is hydroxy, hydroxy-$C_{1-8}$alkyl, benzyloxy, phosphate or carboxypropionyloxy; $R^5$ is hydrogen, $C_{1-8}$alkyl or hydroxy-$C_{1-8}$alkyl; and physiologically acceptable esters of those compounds wherein $R^2$ is hydroxy or hydroxy-$C_{1-8}$alkyl, $R^4$ is hydroxy, hydroxy-$C_{1-8}$alkyl or carboxypropionyloxy or $R^5$ is hydroxy-$C_{1-8}$alkyl; and physiologically acceptable salts of such compounds of formula (I) and their esters, other than (a) the compound of formula (I) wherein X is oxygen, $R^1$ is amino, $R^2$, $R^3$ and $R^5$ are each hydrogen and $R^4$ is hydroxy, and the benzoate ester and physiologically acceptable salts of said compound and (b) the compound of formula (I) wherein X is oxygen, $R^1$ is amino, $R^2$ is hydroxymethyl, $R^3$ and $R^5$ are each hydrogen and $R^4$ is hydroxy, and physiologically acceptable salts and esters of said compound.

2. Compounds as claimed in claim 1 in the form of their acetate or benzoate esters.

3. Physiologically acceptable esters (other than the benzoate ester) of 2-amino-9-(2-hydroxyethoxymethyl)-9H-purine.

4. A process for the preparation of compounds of formula (I) and physiologically acceptable salts and esters thereof as claimed in claim 1 which comprises:

a) deblocking a compound of formula

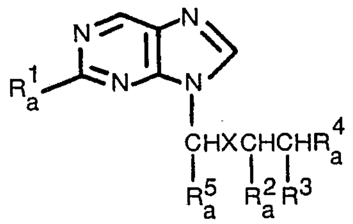

(II)

(wherein X and $R^3$ are as defined in claim 1; $R_a^1$ represents an amino or blocked amino group; $R_a^2$ and $R_a^4$, which may be the same or different, each represents a hydroxy or hydroxyalkyl group or a blocked hydroxy or hydroxyalkyl group, $R_a^5$ represents a hydroxyalkyl or blocked hydroxyalkyl group, providing at least one of $R_a^1$, $R_a^2$, $R_a^4$ and $R_a^5$ represents a blocked group);

b) converting a compound of formula (III)

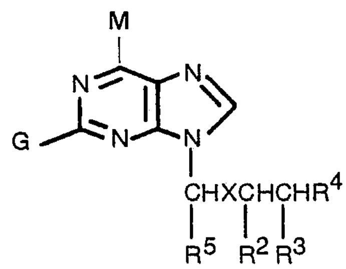

(III)

(wherein G represents

(i) an azide group by catalytic hydrogenation of the azide group to an amino group; or

(ii) a halogen atom or an alkylthio or alkylsulphonyl group by aminolysis of the group using ammonia; and where M in formula (III) represents a halogen atom or a mercapto or thio group by conversion of this group into hydrogen atom);

c) reacting a compound of formula

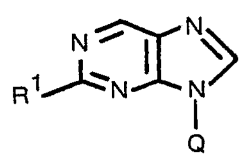

(IV)

(wherein $R^1$ is as defined in claim 1 and Q represents a leaving atom or group) with a compound of formula

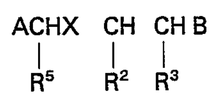

(V)

(wherein X, $R^2$, $R^3$ and $R^5$ are as defined in claim 1, and A represents a leaving group or atom and B represents an optionally protected hydroxy group);

d) reducing a compound of formula

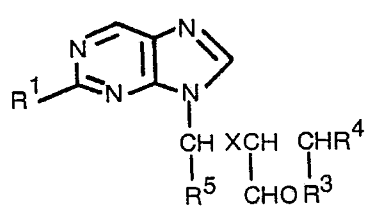

(VI)

(wherein X, $R^1$, $R^3$, $R^4$ and $R^5$ are as defined in claim 1) or a salt or ester thereof to form a compound of formula (I) (wherein $R^2$ represents a hydroxymethyl group) or a physiologically acceptable salt or ester thereof;

e) treatment of a compound of formula

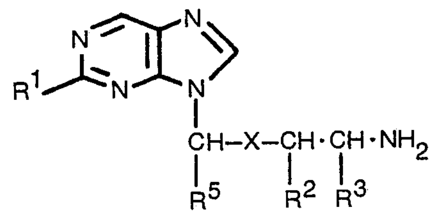

(VII)

(wherein X, $R^1$, $R^2$, $R^3$ and $R^5$ are as defined above) with a nitrite in an acidic medium to form a corresponding compound of formula (I) wherein $R^4$ represents a hydroxy group;
to form a compound of formula (I) or a physiologically acceptable salt or ester thereof, and optionally effecting one or more of the following conversions, in any desired sequence:

i) where the resulting product is a base, converting the said base into a physiologically acceptable acid addition salt thereof;

ii) where the resulting product is an acid addition salt, converting the said salt into the parent base;

iii) where the resulting product is a compound of formula (I) or a salt thereof converting the said compound or salt thereof into a physiologically acceptable ester of the said compound or salt; and/or

iv) where the resulting product is an ester of a compound of formula (I) converting the said ester into the parent compound of formula (I), a physiologically acceptable salt thereof or a different physiologically acceptable ester thereof.

5. A process as claimed in claim 4a) which comprises deblocking a compound of formula

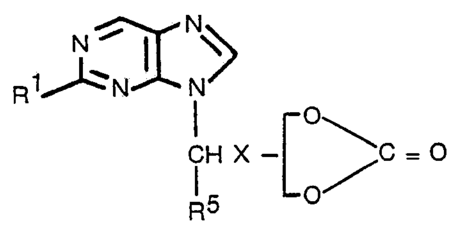

(VIII)

(wherein X, $R^1$ and $R^5$ are as defined in claim 1) to form a compound of formula (I) wherein $R^2$ represents a hydroxyalkyl group, $R^3$ represents a hydrogen atom, and $R^4$ represents a hydroxy group.

6. Pharmaceutical formulations comprising at least one compound as claimed in any of claims 1 to 3 together with one or more pharmaceutically acceptable carriers therefor.

7. Pharmaceutical formulations comprising 2-amino-9-(2-hydroxy-1-hydroxymethylethoxy)methyl-9H-purine together with one or more pharmaceutically acceptable carriers therefor.

8. Compounds as claimed in any of claims 1 to 3 for use in the treatment or prophylaxis of a viral disease in an animal.

9. 2-Amino-9-(2-hydroxy-1-hydroxymethylethoxy)methyl-9H-purine for use in the treatment or prophylaxis of a viral disease in an animal.

**Patentansprüche**

1. Verbindungen der allgemeinen Formel

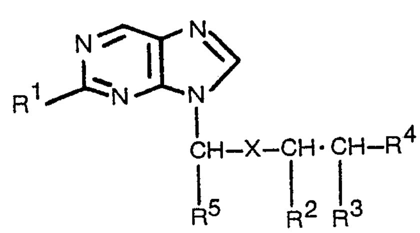

(I)

(worin X Schwefel oder Sauerstoff oder eine chemische Bindung bedeutet; $R^1$ Halogen, Amino oder Azido bedeutet; $R^2$ Wasserstoff, Hydroxy, $C_{1-8}$ Alkyl oder Hydroxy-$C_{1-8}$ Alkyl bedeutet; $R^3$ Wasserstoff oder $C_{1-8}$ Alkyl bedeutet; $R^4$ Hydroxy-$C_{1-8}$ Alkyl, Benzyloxy, Phosphat oder Carboxypropionyloxy bedeutet; $R^5$ Wasserstoff, $C_{1-8}$ Alkyl oder Hydroxy-$C_{1-8}$ Alkyl bedeutet und physiologisch annehmbare Ester von den Verbindungen, worin $R^2$ Hydroxy oder Hydroxy-$C_{1-8}$ Alkyl, $R^4$ Hydroxy, Hydroxy-$C_{1-8}$ Alkyl oder Carboxypropionyloxy bedeutet oder $R^5$ Hydroxy-$C_{1-8}$ Alkyl bedeutet; sowie physiologisch annehmbare Salze von Verbindungen der Formel (I) und ihrer Ester, mit Ausnahme von (a) der Verbindung der Formel (I), worin X Sauerstoff, $R^1$ Amino, $R^2$, $R^3$ und $R^5$ jeweils Wasserstoff und $R^4$ Hydroxy bedeuten und die Benzoatester und physiologisch annehmbare Salze der Verbindung und (b) der Verbindung der Formel (I)

worin X Sauerstoff, $R^1$ Amino, $R^2$ Hydroxymethyl, $R^3$ und $R^5$ jeweils Wasserstoff und $R^4$ Hydroxy bedeuten und physiologisch annehmbare Salze und Ester dieser Verbindung.

2. Verbindung nach Anspruch 1 in Form ihrer Acetat- oder Benzoatester.

3. Physiologisch annehmbare Ester (mit Ausnahme der Benzoatester) von 2-Amino-9-(2-hydroxyethoxymethyl)-9H-purin.

4. Verfahren zur Herstellung von Verbindungen der Formel (I) und physiologisch annehmbarer Salze und Ester davon gemäß Anspruch 1, bei dem man:

a) eine Verbindung der folgenden Formel entblockt

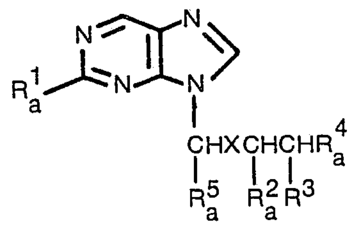

(II)

(worin X und $R^3$ die in Anspruch 1 gegebene Bedeutung besitzen; $R^1_a$ eine Amino- oder eine blockierte Aminogruppe bedeutet; $R^2_a$ und $R^4_a$, die gleich oder verschieden sein können, jeweils eine Hydroxy- oder eine Hydroxyalkylgruppe, oder eine blockierte Hydroxy- oder Hydroxy-alkylgruppe bedeuten; $R^5_a$ eine Hydroxyalkyl oder blockierte Hydroxy-alkylgruppe bedeutet, mit der Maßgabe, daß mindestens einer der Reste von $R^1_a$, $R^2_a$, $R^4_a$ und $R^5_a$ eine blockierte Gruppe bedeutet);

b) eine Verbindung der Formel (III) umsetzt

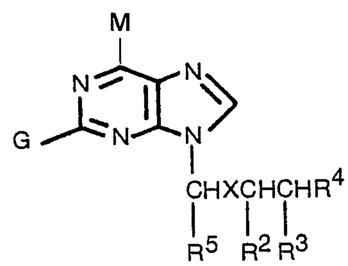

(III)

(worin G bedeutet

i) eine Azidgruppe, durch katalytische Hydrierung der Azidgruppe in eine Aminogruppe; oder

ii) ein Halogenatom oder eine Alkylthio- oder Alkylsulphonylgruppe, durch Aminolyse der Gruppe unter Verwendung von Ammoniak;

und worin M in der Formel (III) ein Halogenatom oder eine Mercapto- oder Thiogruppe bedeutet durch Überführung dieser Gruppe in ein Wasserstoffatom);

c) und eine Verbindung der folgenden Formel

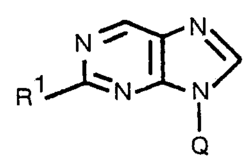

(IV)

(worin $R^1$ die in Anspruch 1 gegebene Bedeutung aufweist und Q ein austretendes Atom oder Gruppe bedeutet) mit einer Verbindung der Formel (V) umsetzt,

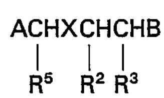

(V)

(worin X, $R^2$, $R^3$ und $R^5$ die in Anspruch 1 gegebene Bedeutung aufweisen und A eine austretende Gruppe oder ein Atom bedeutet und B eine gegebenenfalls geschützte Hydroxygruppe bedeutet);

d) eine Verbindung der Formel (VI)

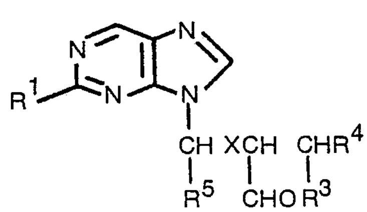

(VI)

(worin X, $R^1$, $R^3$, $R^4$ und $R^5$ die in Anspruch 1 gegebene Bedeutung aufweisen) oder ein Salz oder einen Ester davon reduziert um eine Verbindung der Formel (I) (worin $R^2$ eine Hydroxymethylgruppe bedeutet) oder ein physiologisch annehmbares Salz oder Ester davon), herzustellen;

e) eine Verbindung der Formel (VII)

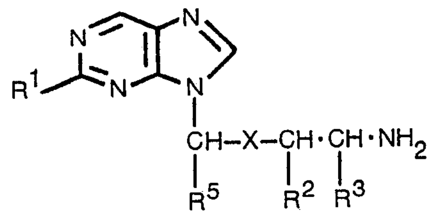

(VII)

(worin X, $R^1$, $R^2$, $R^3$ und $R^5$ die vorhin gegebene Bedeutung aufweisen) mit einem Nitrit in einem sauren Medium behandelt um die entsprechende Verbindung der Formel (I) herzustellen, worin $R^4$ eine Hydroxygruppe bedeutet;

zur Herstellung einer Verbindung der Formel (I) oder eines annehmbaren Salzes oder Ester davon und gegebenenfalls Durchführung einer oder mehrerer der folgenden Umwandlungen in jeder gewünschten Reihenfolge:

i) wenn das erhaltene Produkt eine Base ist, Überführung der Base in ein physiologisch annehmbares Säureadditionssalz;

ii) wenn das erhaltene Produkt ein Säureadditionssalz ist, Umwandlung des Salzes in die Stammbase;

iii) wenn das erhaltene Produkt eine Verbindung der Formel (I) oder ein Salz davon ist Umwandlung der Verbindung oder des Salzes davon in einen physiologisch annehmbaren Ester der Verbindung oder das Salz; und/oder

iv) wenn das erhaltene Produkt ein Ester einer Verbindung der Formel (I) ist, Umwandlung des Esters in die Stammverbindung der Formel (I), ein physiologisch annehmbares Salz davon oder einen verschiedenen physiologisch annehmbaren Ester davon.

5. Verfahren nach Anspruch 4a) bei dem man eine Verbindung der Formel entblockt

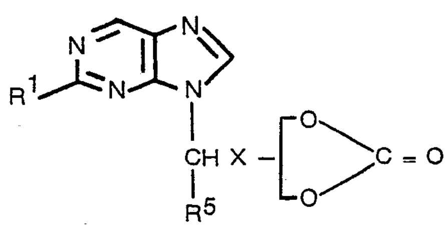

(VIII)

(worin X, $R^1$ und $R^5$ die in Anspruch 1 gegebene Bedeutung aufweisen, um eine Verbindung der Formel (I) herzustellen, worin $R^2$ eine Hydroxyalkylgruppe, $R^3$ eine Wasserstoffatom und $R^4$ eine Hydroxygruppe bedeuten).

6. Pharmazeutisch Formulierung enthaltend mindestens eine Verbindung nach einem der Ansprüche 1 bis 3 in Verbindung mit einem oder mehreren pharmazeutisch annehmbaren Trägermaterialien dafür.

7. Pharmazeutische Formulierung enthaltend 2-Amino-9(2-hydroxy-1-hydroxymethylethoxy)methyl-9H-purin mit einem oder mehreren pharmazeutisch annehmbaren Trägermaterialien dafür.

8. Verbindungen nach einem der Ansprüche 1 bis 3 zur Verwendung in der Behandlung oder Prophylaxe einer viralen Erkrankung bei einem Tier.

9. 2-Amino-9(2-hydroxy-1-hydroxymethylethoxy)methyl-9H-purin zur Verwendung in der Behandlung oder Prophylaxe einer viralen Erkrankung bei einem Tier.

**Revendications**

1. Composés de formule générale

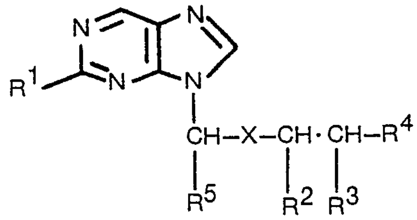

(I)

(où X est soufre ou oxygène ou une liaison chimique;

$R^1$ est halogène, amino ou azido;
$R^2$ est hydrogène, hydroxyle, $C_{1-8}$alcoyle ou hydroxy-$C_{1-8}$alcoyle;
$R^3$ est hydrogène ou $C_{1-8}$alcoyle;
$R^4$ est hydroxyle, hydroxy-$C_{1-8}$alcoyle, benzyloxy, phosphate ou carboxypropionyloxy;
$R^5$ est hydrogène, $C_{1-8}$alcoyle ou hydroxy-$C_{1-8}$alcoyle);
et les esters physiologiquement acceptables de ces composés où $R^2$ est hydroxyle ou hydroxy-$C_{1-8}$alcoyle, $R^4$ est hydroxyle, hydroxy-$C_{1-8}$alcoyle ou carboxypropionyloxy ou $R^5$ est hydroxy-$C_{1-8}$alcoyle;
et les sels physiologiquement acceptables de ces composés de formule (I) et leurs esters, à l'exception

(a) du composé de formule (I) où X est oxygène, $R^1$ est amino, $R^2$, $R^3$ et $R^5$ sont chacun hydrogène et $R^4$ est hydroxyle et de l'ester benzoïque et des sels physiologiquement acceptables de ce composé, et

(b) du composé de formule (I) où X est oxygène, $R^1$ est amino, $R^2$ est hydroxyméthyle, $R^3$ et $R^5$ sont chacun hydrogène et $R^4$ est hydroxyle et des sels et esters physiologiquement acceptables de ce composé.

2. Composés suivant la revendication 1 sous la forme de leurs esters acétiques ou benzoïques.

3. Esters physiologiquement acceptables (autres que l'ester benzoïque) de la 2-amino-9-(2-hydroxyéthoxyméthyl)-9H-purine.

4. Procédé de préparation de composés de formule (I) et de leurs sels et esters physiologiquement acceptables suivant la revendication 1, qui comprend:

a) le déblocage d'un composé de formule

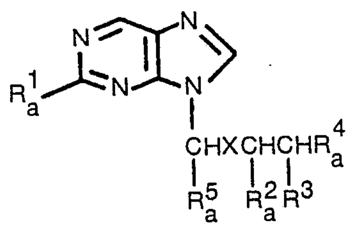

(II)

(où X et $R^3$ sont tels que définis dans la revendication 1; $R^1_a$ représente un radical amino ou amino bloqué $R^2_a$ et $R^4_a$, qui peuvent être identiques ou différents, représentent chacun un radical hydroxyle ou hydroxyalcoyle ou un radical hydroxyle ou hydroxyalcoyle bloqué; $R^5_a$ représente un radical hydroxyalcoyle ou hydroxyalcoyle bloqué,
étant entendu qu'au moins l'une d'entre $R^1_a$, $R^2_a$, $R^4_a$ et $R^5_a$ représente un radical bloqué);

b) la conversion d'un composé de formule (III)

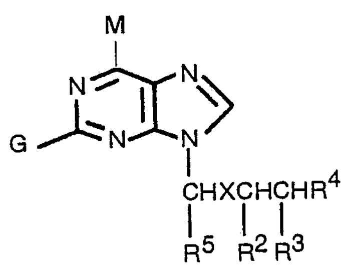

(III)

(où G représente

(i) un radical azido, par hydrogénation catalytique du radical azido en un radical amino, ou

(ii) un atome d'halogène ou un radical alcoylthio ou alcoylsulfonyle, par aminolyse du radical au moyen d'ammoniac;

et lorsque M représente dans la formule (III) un atome d'halogène ou un radical mercapto ou thio, par conversion de ce radical en un atome d'hydrogène);

c) la réaction d'un composé de formule

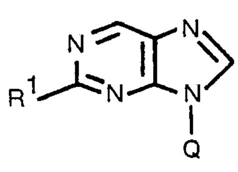

(IV)

(où $R^1$ est tel que défini dans la revendication 1 et Q représente un atome ou radical partant) avec un composé de formule

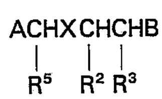

(V)

(où X, $R^2$, $R^3$ et $R^5$ sont tels que définis dans la revendication 1 et A représente un radical ou atome partant et B représente un radical hydroxyle éventuellement protégé);

d) la réduction d'un composé de formule

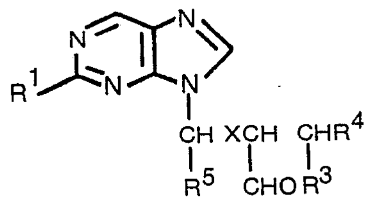

(VI)

(où X, $R^1$, $R^3$, $R^4$ et $R^5$ sont tels que définis dans la revendication 1) ou d'un sel ou ester de celui-ci pour la formation d'un composé de formule (I) (où $R^2$ représente un radical hydroxyméthyle) ou d'un sel ou ester physiologiquement acceptable de celui-ci;

e) le traitement d'un composé de formule

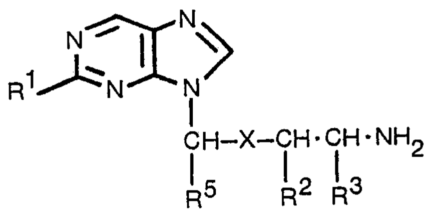

(VII)

(où X, $R^1$, $R^2$, $R^3$ et $R^5$ sont tels que définis ci-dessus) avec un nitrite dans un milieu acide pour la formation du composé correspondant de formule (I) où $R^4$ représente un radical hydroxyle;

pour former un composé de formule (I) ou un sel ou ester physiologiquement acceptable de celui-ci et éventuellement l'exécution d'une ou plusieurs des conversions ci-après dans tout ordre désiré:

i) lorsque le produit résultant est une base, la conversion de cette base en un sel d'addition d'acide physiologiquement acceptable de celle-ci;

ii) lorsque le produit résultant est un sel d'addition d'acide, la conversion de ce sel en la base dont il est issu;

iii) lorsque le produit résultant est un composé de formule (I) ou un sel de celui-ci, la conversion de ce composé ou de son sel en un ester physiologiquement acceptable de ce composé ou de son sel; et/ou

iv) lorsque le produit résultant est un ester d'un composé de formule (I), la conversion de cet ester en le composé de formule (I) dont il est issu, en un sel physiologiquement acceptable de celui-ci ou en un ester physiologiquement acceptable différent.

5. Procédé suivant la revendication 4a), qui comprend le déblocage d'un composé de formule

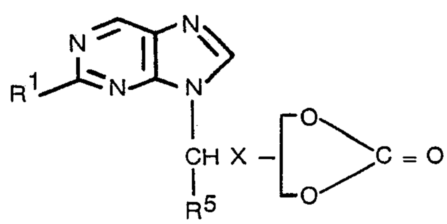

(VIII)

(où X, $R^1$ et $R^5$ sont tels que définis dans la revendication 1)
pour la formation d'un composé de formule (I) où $R^2$ représente un radical hydroxyalcoyle, $R^3$ représente un atome d'hydrogène et $R^4$ représente un radical hydroxyle.

6. Compositions pharmaceutiques comprenant au moins un composé suivant l'une quelconque des revendications 1 à 3 conjointement avec un ou plusieurs excipients pharmaceutiquement acceptables.

7. Compositions pharmaceutiques comprenant de la 2-amino-9-(2-hydroxy-1-hydroxyméthyléthoxy)méthyl-9H-purine conjointement avec un ou plusieurs excipients pharmaceutiquement acceptables.

8. Composés suivant l'une quelconque des revendications 1 à 3, à utiliser pour le traitement ou la prophylaxie d'une affection virale chez un animal.

9. La 2-amino-9-(2-hydroxy-1-hydroxyméthyléthoxy)méthyl-9H-purine, à utiliser pour le traitement ou la prophylaxie d'une affection virale chez un animal.